(19) Europäisches Patentamt
European Patent Office
Office européen des brevets

(11) Veröffentlichungsnummer: **0 330 927**
**A2**

(12) **EUROPÄISCHE PATENTANMELDUNG**

(21) Anmeldenummer: **89102711.2**

(51) Int. Cl.⁴: **C07D 213/81 , A01N 43/40**

(22) Anmeldetag: **17.02.89**

(30) Priorität: **01.03.88 DE 3806489**

(43) Veröffentlichungstag der Anmeldung:
**06.09.89 Patentblatt 89/36**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Wollweber, Detlef, Dr.**
**Paul-Ehrlich-Strasse 17**
**D-5600 Wuppertal 1(DE)**
Erfinder: **Krämer, Wolfgang, Dr.**
**Rosenkranz 25**
**D-5093 Burscheid 2(DE)**
Erfinder: **Dutzmann, Stefan, Dr.**
**Leinenweberweg 33**
**D-4000 Düsseldorf 13(DE)**
Erfinder: **Brandes, Wilhelm, Dr.**
**Eichendorffstrasse 3**
**D-5653 Leichlingen 1(DE)**

(54) **Pyridin-4-carbonsäureanilide.**

(57) Pyridin-4-carbonsäureanilide der allgemeinen Formel (I),

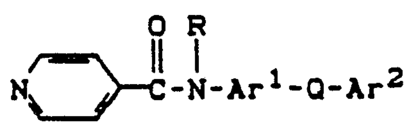

(I)

in welcher
R, Q, $Ar^1$ und $Ar^2$ die in der Beschreibung gegebenen Bedeutungen haben, deren Säureadditionssalze und Metallsalzkomplexe und ihre Verwendung in Schädlingsbekämpfungsmitteln.
Die Pyridin-4-carbonsäureanilide sind neu und durch die Formel (I) definiert. Sie können nach Analogieverfahren hergestellt werden, z.B. indem man ein Pyridin-4-carbonsäurehalogenid mit geeigneten Aminobenzophenonen umsetzt oder geeignete Pyridin-4-carbonsäureanilide reduziert oder geeignete Pyridin-4-carbonsäureanilide alkyliert oder mit geeigneten Alkoholen bzw. Diolen umsetzt und anschließend gegebenenfalls eine Säure oder ein Metallsalz addiert.

EP 0 330 927 A2

## Pyridin-4-carbonsäureanilide

Die Erfindung betrifft neue Pyridin-4-carbonsäureanilide, mehrere Verfahren zu ihrer Herstellung und ihre Verwendung als Schädlingsbekämpfungsmittel.

Es ist bekannt, daß bestimmte Pyridin-4-carbonsäureanilide, wie beispielsweise die Verbindung N-[4-Chlor-2-(4-chlor-α,α-diethoxybenzyl)-phenyl]-pyridin-4-carbonsäureamid oder die Verbindung N-[2-(4-Chlorbenzoyl)-phenyl]-pyridin-4-carbonsäureamid oder die Verbindung N-[2-(4-Chlor-α-hydroxybenzyl)-phenyl]-pyridin-4-carbonsäureamid oder die Verbindung N-[2-(4-Chlor-α,α-diethoxybenzyl)-phenyl]-pyridin-4-carbonsäureamid fungizide Wirksamkeit besitzen (vgl. z.B. EP 122 410; Jpn. Kokai Tokkyo Koho Jp 60/32 703; Jpn. Kokai Tokkyo Koho Jp 60/23 364).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Pyridin-4-carbonsäureanilide der allgemeinen Formel (I),

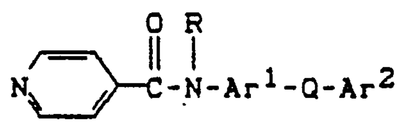

(I)

in welcher
R für Wasserstoff oder Alkyl steht,
Q für einen Rest der Formel

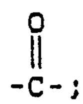 ; 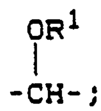 ; 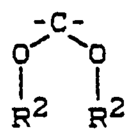

oder

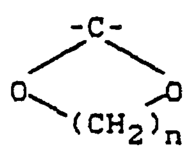

steht,
wobei
$R^1$ für Wasserstoff oder Alkyl steht,
$R^2$ für Alkyl steht und
n für eine Zahl 2, 3 oder 4 steht,
$Ar^1$ für einen gegebenenfalls substituierten o-Phenylenrest steht und
$Ar^2$ für gegebenenfalls substituiertes Phenyl steht,
mit der Maßgabe, daß mindestens einer der Reste $Ar^1$ oder $Ar^2$ substituiert ist durch Halogenalkoxy oder Halogenalkylthio, sowie deren Säureadditionssalze und Metallsalzkomplexe gefunden.

Weiterhin wurde gefunden, daß man die neuen Pyridin-4-carbonsäureanilide der Formel (I),

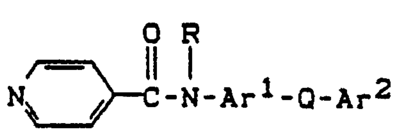

(I)

in welcher
R für Wasserstoff oder Alkyl steht,
Q für einen Rest der Formel

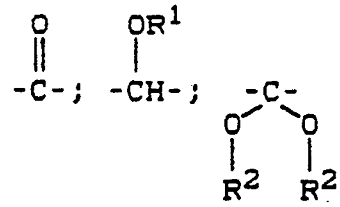

oder

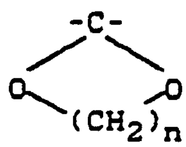

steht,
wobei
$R^1$ für Wasserstoff oder Alkyl steht,
$R^2$ für Alkyl steht und
n für eine Zahl 2, 3 oder 4 steht,
$Ar^1$ für einen gegebenenfalls substituierten o-Phenylenrest steht und
$Ar^2$ für gegebenenfalls substituiertes Phenyl steht,
mit der Maßgabe, daß mindestens einer der Reste $Ar^1$ oder $Ar^2$ substituiert ist durch Halogenalkoxy oder Halogenalkylthio, sowie deren Säureadditionssalze und Metallsalzkomplexe nach einem der im folgenden beschriebenen Verfahren erhält:

(a) Man erhält Pyridin-4-carbonsäureanilide der Formel (Ia),

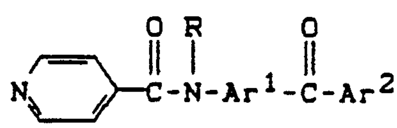

(Ia)

in welcher
R, $Ar^1$ und $Ar^2$ die oben angegebene Bedeutung haben,
wenn man Pyridin-4-carbonsäurehalogenid Hydrochloride der Formel (II),

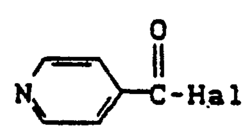

x HCl (II)

in welcher
Hal für Halogen, wie Fluor, Chlor oder Brom, vorzugsweise für Chlor steht,
mit Aminobenzophenonen der Formel (III),

$R-NH-Ar^1-\overset{O}{\overset{||}{C}}-Ar^2$ (III)

in welcher
R, $Ar^1$ und $Ar^2$ die oben angegebene Bedeutung haben,
gegebenenfalls in Gegenwart eines Verdünnungsmittels und gegebenenfalls in Gegenwart eines Säurebindemittels umsetzt;

(b) man erhält Pyridin-4-carbonsäureanilide der Formel (Ib),

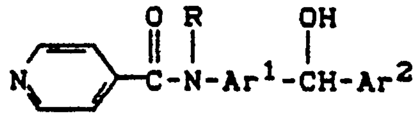 (Ib)

in welcher
R, $Ar^1$ und $Ar^2$ die oben angegebene Bedeutung haben,
wenn man die mit Hilfe des Verfahrens (a) erhältlichen Pyridin-4-carbonsäureanilide der Formel (Ia),

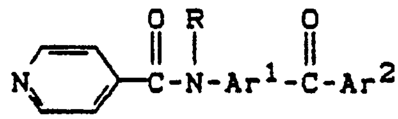 (Ia)

in welcher
R, $Ar^1$ und $Ar^2$ die oben angegebene Bedeutung haben,
mit Reduktionsmitteln gegebenenfalls in Gegenwart eines Verdünnungsmittels umsetzt;

(c) man erhält Pyridin-4-carbonsäureanilide der Formel (Ic),

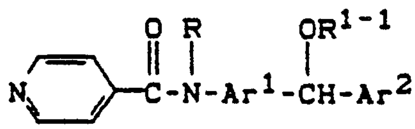 (Ic)

in welcher
$R^{1-1}$ für Alkyl steht und
R, $Ar^1$ und $Ar^2$ die oben angegebene Bedeutung haben,
wenn man die mit Hilfe des Verfahrens (b) erhältlichen Pyridin-4-carbonsäureanilide der Formel (Ib),

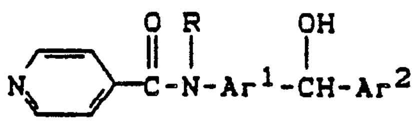 (Ib)

in welcher
R, $Ar^1$ und $Ar^2$ die oben angegebene Bedeutung haben,
mit Alkylierungsmitteln der Formel (IV),

$R^{1-1}$-E (IV)

in welcher
$R^{1-1}$ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt;

(d) man erhält Pyridin-4-carbonsäureanilide der Formel (Id),

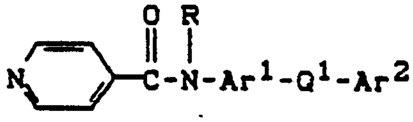 (Id)

in welcher
$Q^1$ für einen Rest der Formel

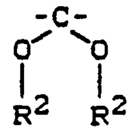

oder

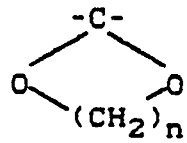

steht, wobei
$R^2$ und n die oben angegebene Bedeutung haben,
wenn man die mit Hilfe des Verfahrens (a) erhältlichen Pyridin-4-carbonsäureanilide der Formel (Ia),

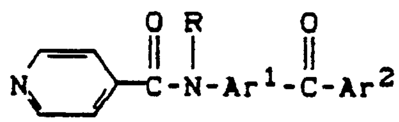 (Ia)

in welcher
R, $Ar^1$ und $Ar^2$ die oben angegebene Bedeutung haben,
mit mindestens 2 Mol Alkohol oder mindestens 1 Mol Diol der Formel (V),
$HO-R^3$ (V)
in welcher
$R^3$ für Alkyl oder für einen Rest $-(CH_2)_n-OH$ steht,
wobei
n die oben angegebene Bedeutung hat,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

Schließlich wurde gefunden, daß die neuen Pyridin-4-carbonsäureanilide der allgemeinen Formel (I) sowie deren Säureadditionssalze und Metallsalzkomplexe eine gute Wirksamkeit gegen Schädlinge besitzen.

Überraschenderweise zeigen die erfindungsgemäßen Pyridin-4-carbonsäureanilide der allgemeinen Formel (I) z.B. eine erheblich bessere fungizide Wirksamkeit als die aus dem Stand der Technik bekannten Pyridin-4-carbonsäureanilide, wie beispielsweise die Verbindung N-[4-Chlor-2-(4-chlor-α,α-diethoxybenzyl)-phenyl]-pyridin-4-carbonsäureamid oder die Verbindung N-[2-(4-Chlorbenzoyl)- phenyl]-pyridin-4-carbonsäureamid oder die Verbindung N-[2-(4-Chlor-α-hydroxybenzyl)-phenyl]-pyridin-4-carbonsäureamid oder die Verbindung N-[2-(4-Chlor-α,α-diethoxybenzyl)-phenyl]-pyridin-4-carbonsäureamid, welche chemisch und wirkungsmäßig naheliegende Verbindungen sind.

Die erfindungsgemäßen Pyridin-4-carbonsäureanilide sind durch die Formel (I) allgemein definiert. Bevorzugt sind Verbindungen der Formel (I), bei welchen
R für Wasserstoff oder für geradkettiges oder verzweigtes Alkyl mit 1 bis 4 Kohlenstoffatomen steht,
Q für einen Rest der Formel

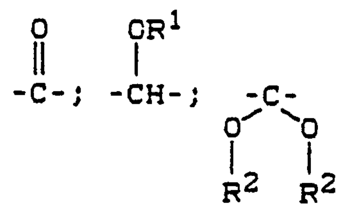

oder

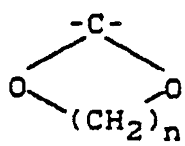

steht,
wobei
$R^1$ für Wasserstoff oder für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen steht und
n für eine Zahl 2, 3 oder 4 steht,
$Ar^1$ für einen gegebenenfalls einfach bis vierfach, gleich oder verschieden substituierten o-Phenylenrest steht und
$Ar^2$ für gegebenenfalls einfach bis fünffach, gleich oder verschieden substituiertes Phenyl steht, wobei als Phenyl- bzw. Phenylensubstituenten für die Reste $Ar^1$ bzw. $Ar^2$ jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
mit der Maßgabe, daß mindestens einer der Reste $Ar^1$ oder $Ar^2$ substituiert ist durch Halogenalkoxy oder Halogenalkylthio.

Besonders bevorzugt sind Verbindungen der Formel (I), bei welchen
R für Wasserstoff, Methyl oder Ethyl steht,
Q für einen Rest der Formel

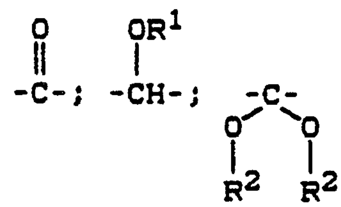

oder

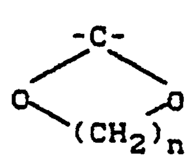

steht,
wobei
$R^1$ für Wasserstoff, Methyl oder Ethyl steht,
$R^2$ für Methyl oder Ethyl steht und
n für eine Zahl 2 oder 3 steht,
$Ar^1$ für einen gegebenenfalls ein- bis dreifach, gleich oder verschieden substituierten o-Phenylenrest steht

und

$Ar^2$ für gegebenenfalls ein- bis dreifach, gleich oder verschieden substituiertes Phenyl steht, wobei als Phenyl- bzw. Phenylensubstituenten für die Reste $Ar^1$ bzw. $Ar^2$ jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Difluormethyl, Difluorchlormethyl, Dichlorfluormethyl, Trifluormethoxy, Trifluormethylthio, Difluormethoxy, Difluormethylthio, Difluorchlormethoxy, Difluorchlormethylthio, Dichlorfluormethoxy oder Dichlorfluormethylthio,

mit der Maßgabe, daß mindestens einer der Reste $Ar^1$ oder $Ar^2$ substituiert ist durch Trifluormethoxy, Trifluormethylthio, Difluormethoxy, Difluormethylthio, Difluorchlormethoxy, Difluorchlormethylthio, Dichlorfluormethoxy oder Dichlorfluormethylthio.

Ganz besonders bevorzugt sind Verbindungen der Formel (I), bei welchen

R für Wasserstoff oder Methyl steht,

Q für einen Rest der Formel

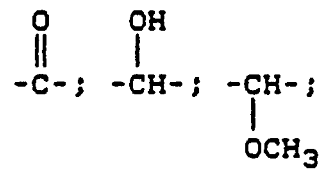

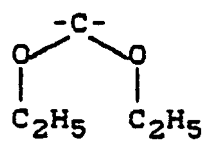

oder

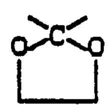

steht,

$Ar^1$ für einen gegebenenfalls ein- bis dreifach, gleich oder verschieden substituierten o-Phenylenrest steht und

$Ar^2$ für gegebenenfalls ein- bis dreifach, gleich oder verschieden substituiertes Phenyl steht, wobei als Phenyl- bzw. Phenylensubstituenten für die Reste $Ar^1$ bzw. $Ar^2$ jeweils infrage kommen: Fluor, Chlor, Methyl, Trifluormethyl, Trifluormethoxy, Difluormethoxy oder Difluorchlormethoxy

mit der Maßgabe, daß mindestens einer der Reste $Ar^1$ oder $Ar^2$ substituiert ist durch Trifluormethoxy, Difluormethoxy oder Difluorchlormethoxy.

Bevorzugte erfindungsgemäße Verbindungen sind auch Additionsprodukte aus Säuren und denjenigen Pyridin-4- carbonsäureaniliden der Formel (I), in denen die Substituenten R, Q, $Ar^1$ und $Ar^2$ die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Pyridin-4-carbonsäureaniliden der Formel (I), in denen die Substituenten R, Q, $Ar^1$ und $Ar^2$ die Bedeutungen haben, die bereits vorzugsweise für diesen Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusam menhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden Pyridin-4-carbonsäureanilide der allgemeinen Formel (I) genannt:

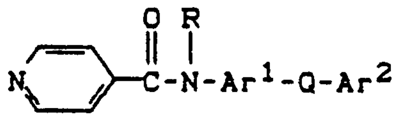 (I)

| R | $Ar^1$ | Q | $Ar^2$ |
|---|---|---|---|
| H | 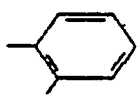 | 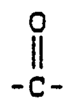 | 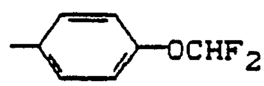 |
| H | 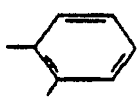 | 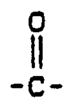 | 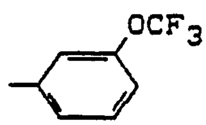 |
| H | 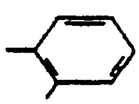 | 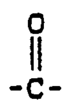 | 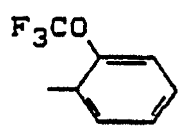 |
| H | 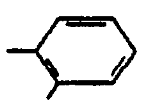 | 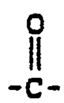 | 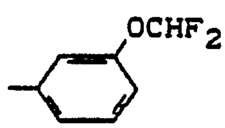 |

| R | $Ar^1$ | Q | $Ar^2$ |
|---|---|---|---|
| H | 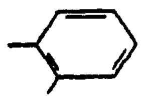 | 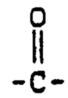 | 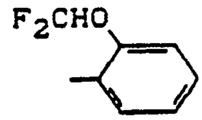 |
| H | 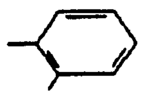 | 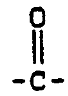 | 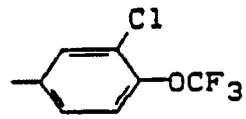 |
| H | 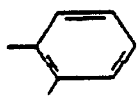 | 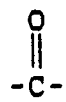 | 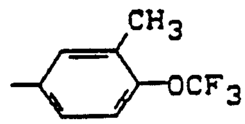 |
| H | 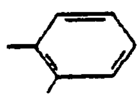 | 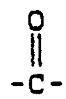 | 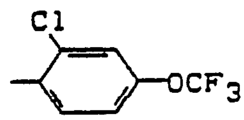 |
| H | 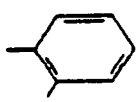 | 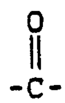 | 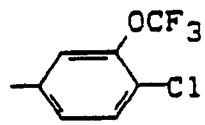 |
| H | 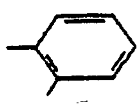 | 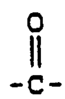 | 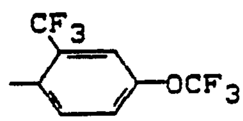 |
| H | 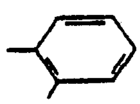 | 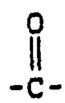 | 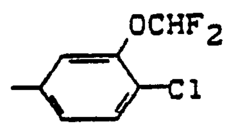 |
| H | 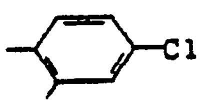 | 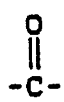 | 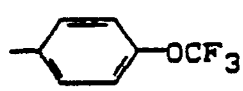 |
| H | 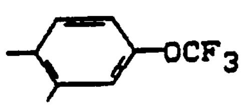 | 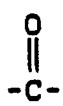 | 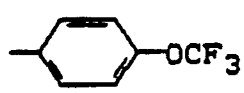 |

| R | $Ar^1$ | Q | $Ar^2$ |
|---|---|---|---|
| H | 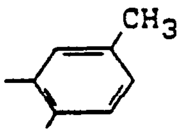 | 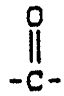 | 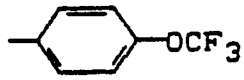 |
| H | 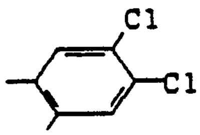 | 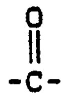 | 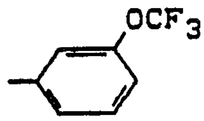 |
| H | 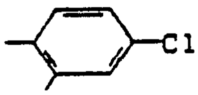 | 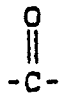 | 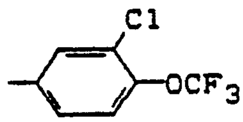 |
| H | 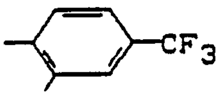 | 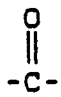 | 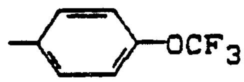 |
| H | 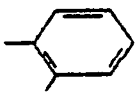 | 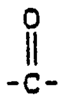 | 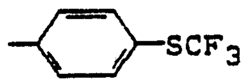 |
| H | 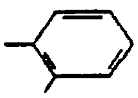 | 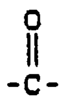 | 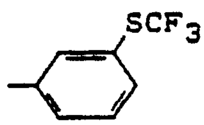 |
| $CH_3$ | 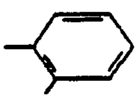 | 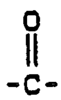 | 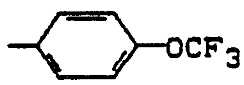 |
| $CH_3$ | 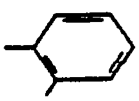 | 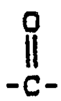 | 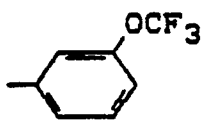 |

| R | $Ar^1$ | Q | $Ar^2$ |
|---|---|---|---|
| $CH_3$ | 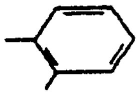 | 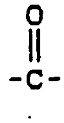 | 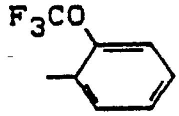 |
| H | 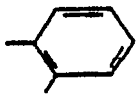 | 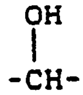 | 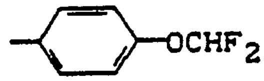 |
| H | 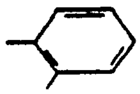 | 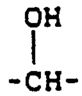 | 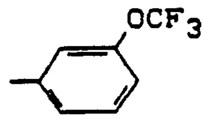 |
| H | 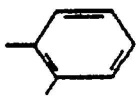 | 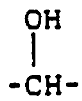 | 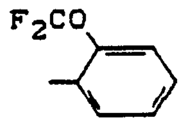 |
| H | 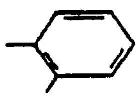 | 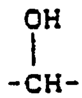 | 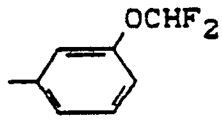 |
| H | 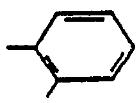 | 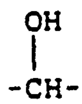 | 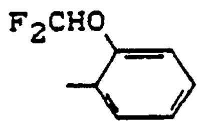 |
| H | 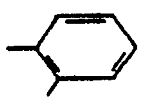 | 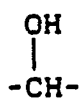 | 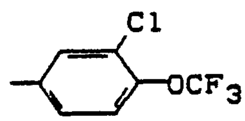 |
| H | 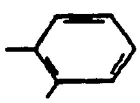 | 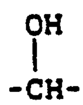 | 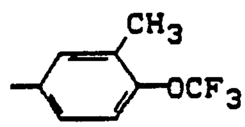 |
| H | 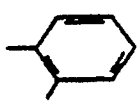 | 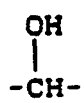 | 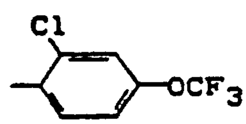 |

| R | $Ar^1$ | Q | $Ar^2$ |
|---|---|---|---|
| H | 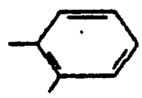 | 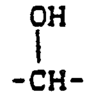 | 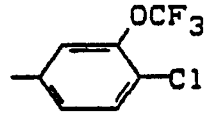 |
| H | 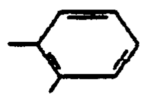 | 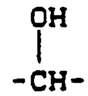 | 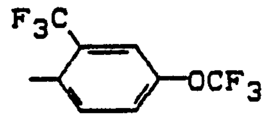 |
| H | 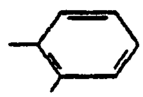 | 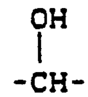 | 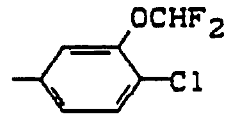 |
| H | 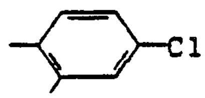 | 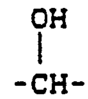 | 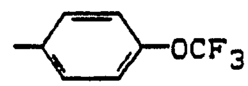 |
| H | 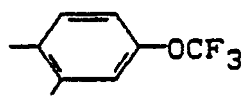 | 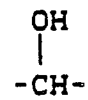 | 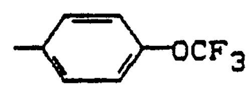 |
| H | 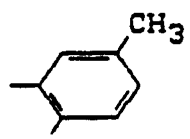 | 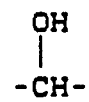 | 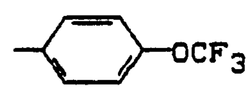 |
| H | 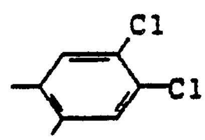 | 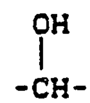 | 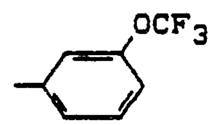 |
| H | 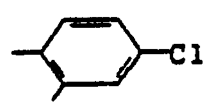 | 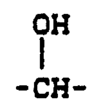 | 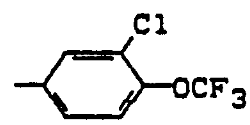 |
| H | 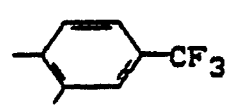 | 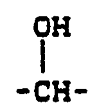 | 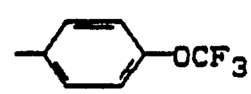 |

| R | $Ar^1$ | Q | $Ar^2$ |
|---|---|---|---|
| H | 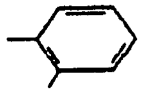 | 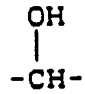 | 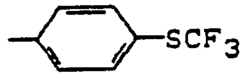 |
| H | 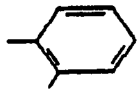 | 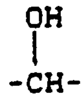 | 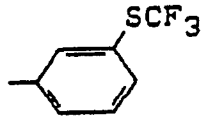 |
| $CH_3$ | 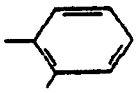 | 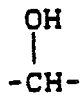 | 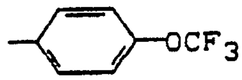 |
| $CH_3$ | 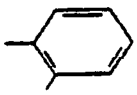 | 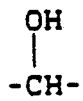 | 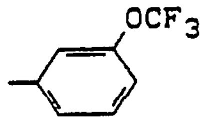 |
| $CH_3$ | 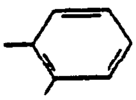 | 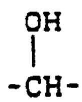 | 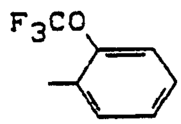 |
| H | 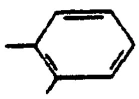 | 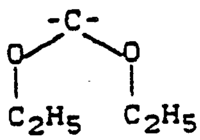 | 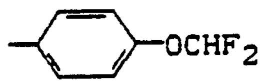 |
| H | 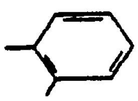 | 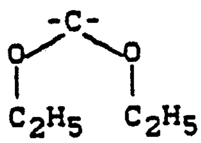 | 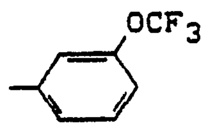 |
| H | 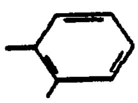 | 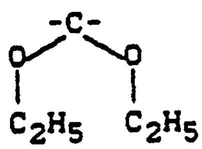 | 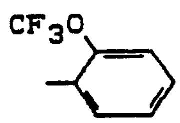 |

| R | $Ar^1$ | Q | $Ar^2$ |
|---|---|---|---|
| H | 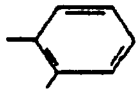 | 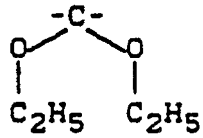 | 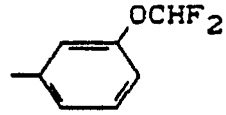 |
| H | 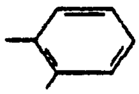 | 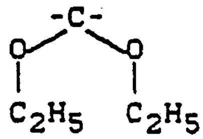 | 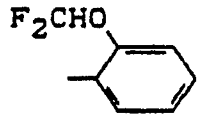 |
| H | 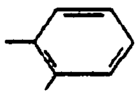 | 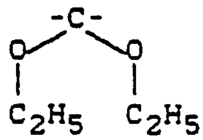 | 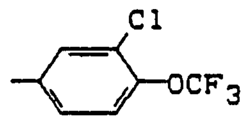 |
| H | 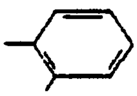 | 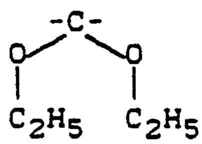 | 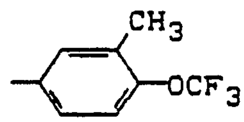 |
| H | 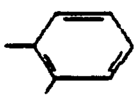 | 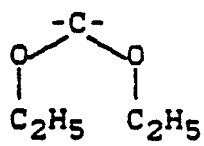 | 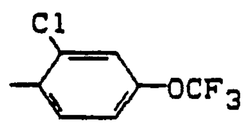 |
| H | 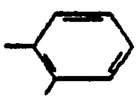 | 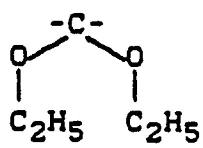 | 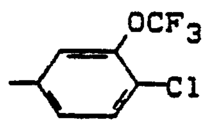 |

| R | $Ar^1$ | Q | $Ar^2$ |
|---|---|---|---|
| H | 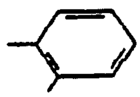 | 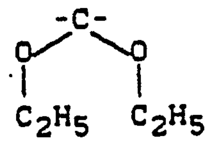 | 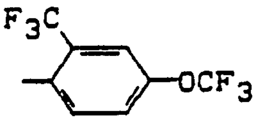 |
| H | 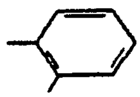 | 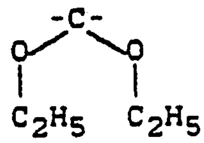 | 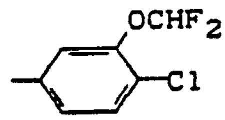 |
| H | 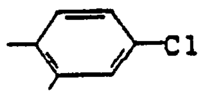 | 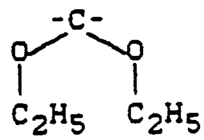 | 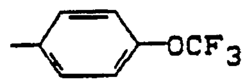 |
| H | 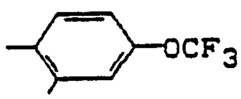 | 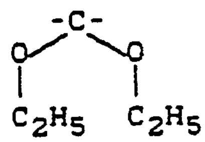 | 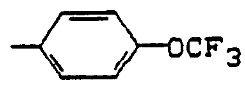 |
| H | 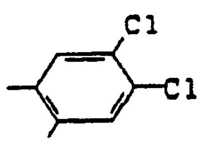 | 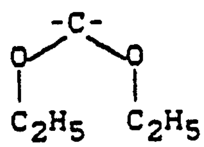 | 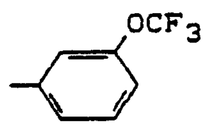 |
| H | 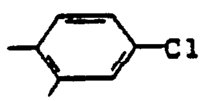 | 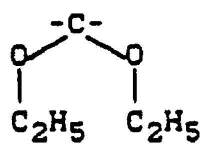 | 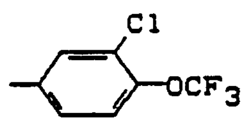 |

| R | $Ar^1$ | Q | $Ar^2$ |
|---|---|---|---|
| H | 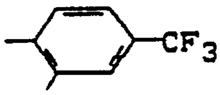 | 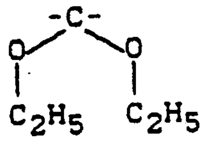 | 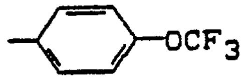 |
| H | 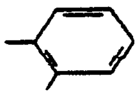 | 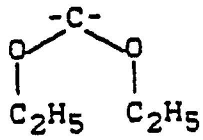 | 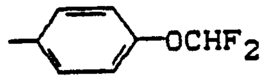 |
| H | 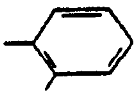 | 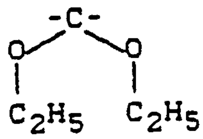 | 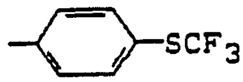 |
| H | 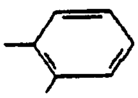 | 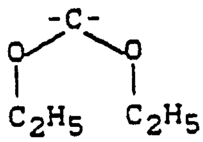 | 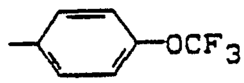 |
| $CH_3$ | 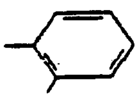 | 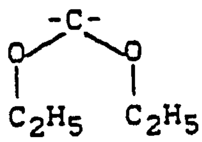 | 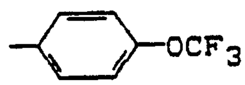 |
| $CH_3$ | 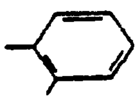 | 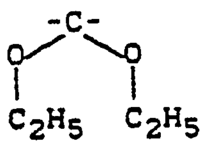 | 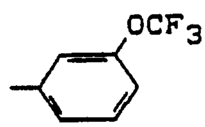 |
| $CH_3$ | 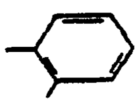 | 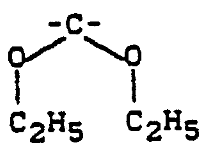 | 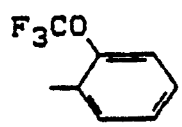 |

| R | $Ar^1$ | Q | $Ar^2$ |
| --- | --- | --- | --- |
| H | 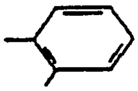 | 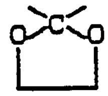 | 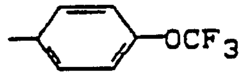 |
| H | 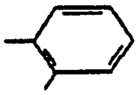 | 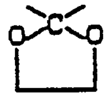 | 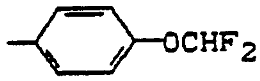 |
| H | 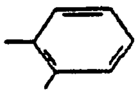 | 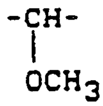 | 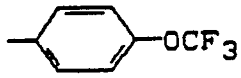 |
| H | 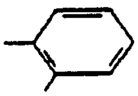 | 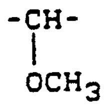 | 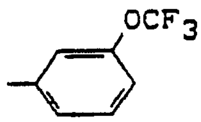 |
| H | 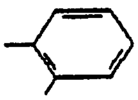 | 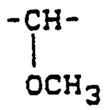 | 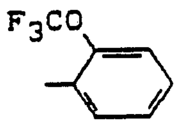 |
| H | 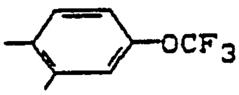 | 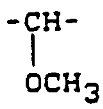 | 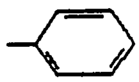 |
| H | 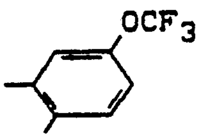 | 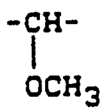 | 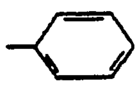 |
| H | 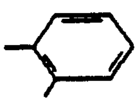 | 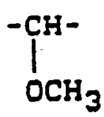 | 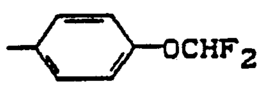 |

| R | $Ar^1$ | Q | $Ar^2$ |
|---|---|---|---|
| H | 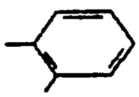 | 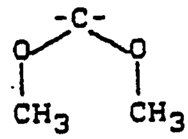 | 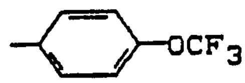 |
| H | 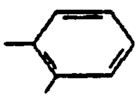 | 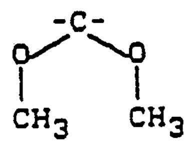 | 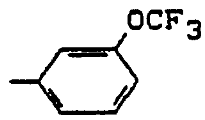 |
| H | 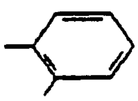 | 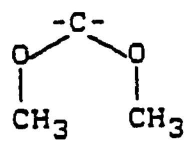 | 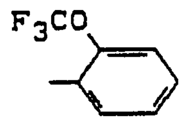 |
| H | 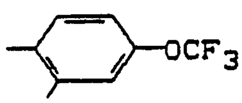 | 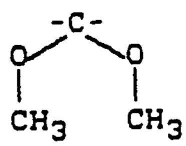 | 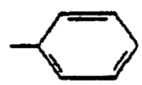 |
| H | 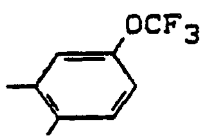 | 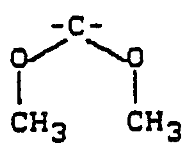 | 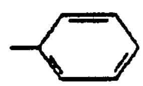 |
| H | 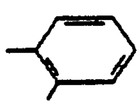 | 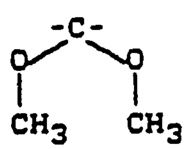 | 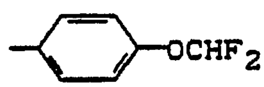 |

Verwendet man beispielsweise Pyridin-4-carbonsäurechlorid Hydrochlorid und 2-Amino-4′-trifluormethoxybenzophenon als Ausgangsstoffe, so läßt sich der Reaktionsablauf des erfindungsgemäßen Verfahrens (a) durch das folgende Formelschema darstellen:

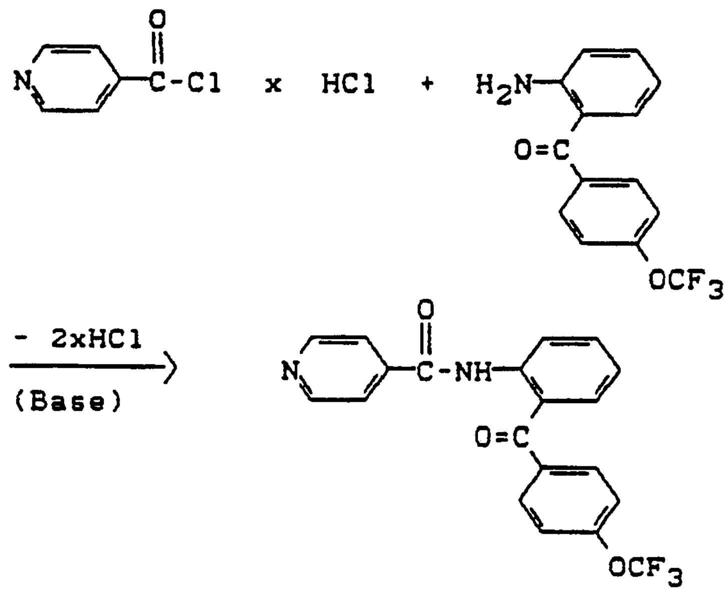


Verwendet man beispielsweise N-[2-(4-Trifluormethoxybenzoyl)-phenyl]-pyridin-4-carbonsäureamid als Ausgangsverbindung und Natriumborhydrid als Reduktionsmittel, so läßt sich der Reaktionsablauf des erfindungsgemäßen Verfahrens (b) durch das folgende Formelschema darstellen:

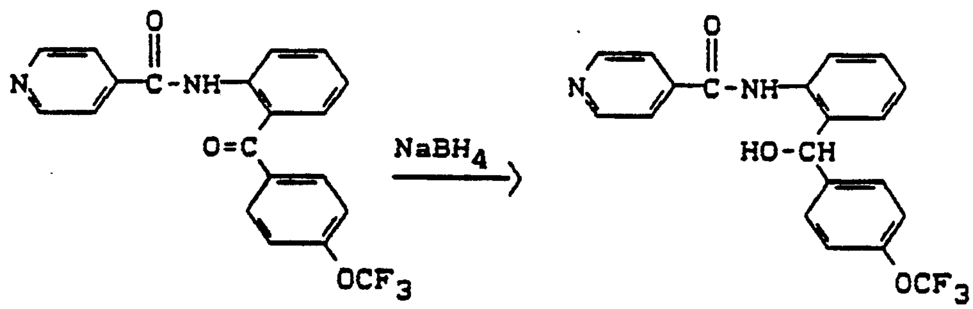


Verwendet man beispielsweise N-[2-(4-Trifluormethoxy-α-hydroxybenzyl)-phenyl]-pyridin-4-carbonsäureamid und Dimethylsulfat als Ausgangsstoffe, so läßt sich der Reaktionsablauf des erfindungsgemäßen Verfahrens (c) durch das folgende Formelschema darstellen:

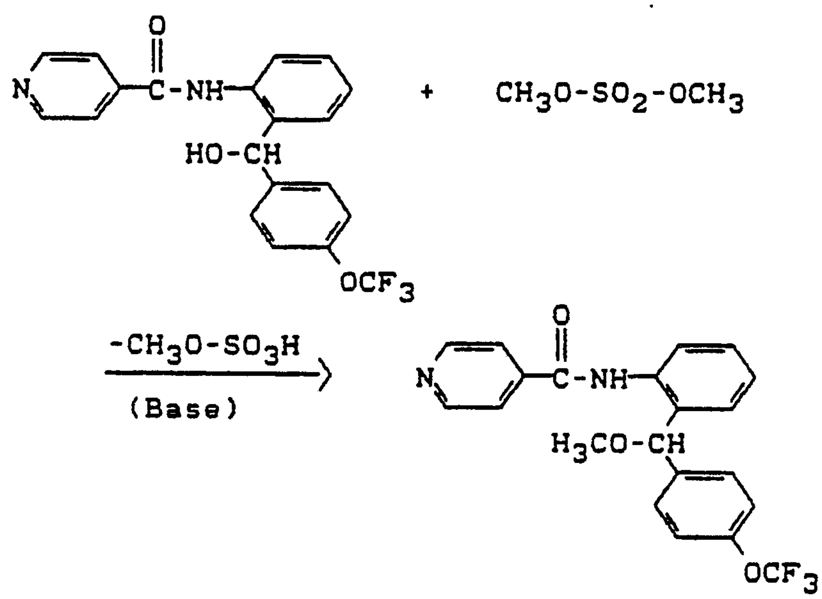


Verwendet man beispielsweise N-[2-(4-Trifluormethoxybenzoyl)-phenyl]-pyridin-4-carbonsäureamid und Ethanol als Ausgangsstoffe, so läßt sich der Reaktionsablauf des erfindungsgemäßen Verfahrens (d) durch das folgende Formelschema darstellen:

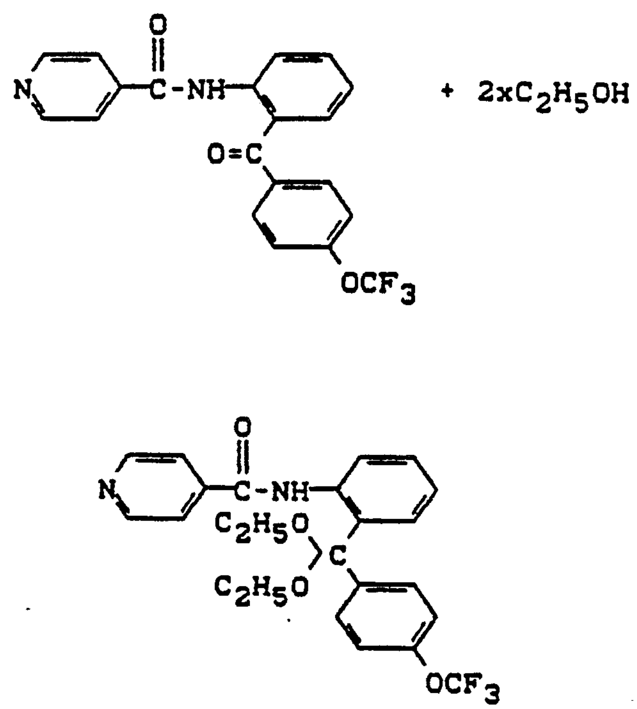


Die zur Durchführung des erfindungsgemäßen Verfahrens (a) als Ausgangsstoffe benötigten Pyridin-4-carbonsäurehalogenid Hydrochloride sind durch die Formel (II) definiert.

Die Pyridin-4-carbonsäurehalogenid Hydrochloride der Formel (II) sind bekannt (vgl. z.B. J. org. Chem. 47, 2633-2637 [1982]; US-PS 38 13 400).

Die zur Durchführung des erfindungsgemäßen Verfahrens (a) weiterhin als Ausgangsstoffe benötigten Aminobenzophenone sind durch die Formel (III) allgemein definiert. In dieser Formel (III) stehen R, $Ar^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Aminobenzophenone der Formel (III) sind teilweise bekannt (vgl. z.B. Khim.-Farm. Zh. 15, 28-31 [1981] bzw. CA 96 : 97 054p; Khim. Geterotsikl. Soedin. 1975, 268-272 bzw. CA 82: 170 851p; Fiziol. Akt.

Veshchestva 14, 36-39 [1982] bzw. CA 99: 88 147f; FR.M. FR 7666 vom 09.02.1970 bzw. CA 76: 12 70 289) oder erhältlich in Analogie zu bekannten Verfahren, beispielsweise wenn man Aniline der Formel (VI),

$H_2N$-$Ar^1$-H (VI)

in welcher

$Ar^1$ die oben angegebene Bedeutung hat,

mit Benzonitrilen der Formel (VII),

NC-$Ar^2$ (VII)

in welcher

$Ar^2$ die oben angegebene Bedeutung hat,

gegebenenfalls in Gegenwart eines Verdünnungsmittels, wie beispielsweise Dichlormethan oder 1,2-Dichlorethan, sowie in Gegenwart eines geeigneten Reaktionshilfsmittels, wie beispielsweise ein Gemisch aus Bortrichlorid und Aluminiumtrichlorid, bei Temperaturen zwischen -5 °C und + 120 °C umsetzt (vgl. hierzu auch J. Amer. chem. Soc. 100, 4842 [1978] sowie die Herstellungsbeispiele).

Aniline der Formel (VI) und Benzonitrile der Formel (VII) sind allgemein bekannte Verbindungen der organischen Chemie (vgl. z.B. US-PS 44 34 182; EP 65 447; DE-OS 28 12 169; Zh. Obshch. Khim. 39, 206-210 [1969] bzw. CA 70: 96 318d; J. org. Chem. 44, 2907-2910 [1979]).

Die zur Durchführung der erfindungsgemäßen Verfahren (b) und (d) als Ausgangsstoffe benötigten Pyridin-4-carbonsäureanilide sind durch die Formel (Ia) allgemein definiert. In dieser Formel (Ia) stehen R, $Ar^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Pyridin-4-carbonsäureanilide der Formel (Ia) sind erfindungsgemäße Verbindungen und erhältlich mit Hilfe des erfindungsgemäßen Verfahrens (a).

Die zur Durchführung des erfindungsgemäßen Verfahrens (c) als Ausgangsstoffe benötigten Pyridin-4-carbonsäureanilide sind durch die Formel (Ib) allgemein definiert. In dieser Formel (Ib) stehen R, $Ar^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Pyridin-4-carbonsäureanilide der Formel (Ib) sind erfindungsgemäße Verbindungen und erhältlich mit Hilfe des erfindungsgemäßen Verfahrens (b).

Die zur Durchführung des erfindungsgemäßen Verfahrens (c) weiterhin als Ausgangsstoffe benötigten Alkylierungsmittel sind durch die Formel (IV) allgemein definiert. In dieser Formel (IV) steht $R^{1-1}$ vorzugsweise für geradkettiges oder verzweigtes Alkyl mit 1 bis 4 Kohlenstoffatomen, insbesondere für Methyl oder Ethyl.

E steht für eine bei Alkylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest.

Die Alkylierungsmittel der Formel (IV) sind allgemein bekannte Verbindungen der organischen Chemie.

Die zur Durchführung des erfindungsgemäßen Verfahrens (d) weiterhin als Ausgangsstoffe benötigten Alkohole oder Diole sind durch die Formel (V) allgemein definiert. In dieser Formel (V) steht $R^3$ vorzugsweise für geradkettiges oder verzweigtes Alkyl mit 1 bis 4 Kohlenstoffatomen, insbesondere für Methyl oder Ethyl oder für einen Rest $-(CH_2)_n-OH$, wobei n vorzugsweise für eine Zahl 2, 3 oder 4, insbesondere für eine Zahl 2 oder 3 steht.

Die Alkohole oder Diole der Formel (V) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Basen, wie Pyridin.

Das erfindungsgemäße Verfahren (a) wird vorzugsweise in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in

einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (a) setzt man pro Mol an Pyridin-4-carbonsäurehalogenid Hydrochlorid der Formel (III) im allgemeinen 0.8 bis 3.0 Mol, vorzugsweise 1.0 bis 1.5 Mol an Aminobenzophenon der Formel (III) und gegebenenfalls 1.0 bis 20.0 Mol, vorzugsweise 1.0 bis 10.0 Mol an Säurebindemittel ein.
Die Reaktionsdurchführung, Aufarbeitung und Isolierung der Reaktionsprodukte erfolgt nach allgemein üblichen Methoden (vgl. auch die Herstellungsbeispiele).

Als Reduktionsmittel zur Durchführung des erfindungsgemäßen Verfahrens (b) kommen alle für derartige Carbonylgruppen-Reduktionen üblichen Reduktionsmittel infrage. Mit besonderem Vorzug verwendet man komplexe Hydride, wie Natriumborhydrid, Natriumcyanoborhydrid oder Lithiumborhydrid, gegebenenfalls in Gegenwart von Calciumchlorid, wobei sich im Reaktionsgemisch auch komplexe Calciumborhydride bilden können.

Als Verdünnungsmittel zur Durchführung des erfindungsgemäßen Verfahrens (b) kommen inerte organische Lösungsmittel infrage. Vorzugsweise verwendet man Ether, wie Diethylether, Dioxan, Tetrahydrofuran oder Ethylenglykoldimethyl- oder -diethylether oder Alkohole, wie Methanol, Ethanol, n- oder i-Propanol gegebenenfalls auch in Mischung mit Wasser.

Die Reaktionstemperaturen können bei der Durchführung des erfindungsgemäßen Verfahrens (b) in einem größeren Bereich variiert werden. Im allgemeinen arbeitet man bei Temperaturen zwischen - 100 °C und + 200 °C, vorzugsweise bei Temperaturen zwischen - 50 °C und + 50 °C.

Zur Durchführung des erfindungsgemäßen Verfahrens (b) setzt man pro Mol an Pyridin-4-carbonsäureanilid der Formel (Ia) im allgemeinen 0.1 bis 1.5 Mol, vorzugsweise 0.25 bis 1.0 Mol an komplexem Hydrid und gegebenenfalls 0.1 bis 1.5 Mol, vorzugsweise 0.25 bis 1.0 Mol an Calciumchlorid ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N- Methylpyrrolidon oder Hexamethylphosphorsäuretriamid, Ester, wie Essigsäureethylester oder Sulfoxide, wie Dimethylsulfoxid.

Das erfindungsgemäße Verfahren (c) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}$/$C_{15}$-alkylammoniumchlorid, Dibenzyl-dimethylammoniummethylsulfat, Dimethyl-$C_{12}$/$C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Das erfindungsgemäße Verfahren (c) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen - 50 °C und 200 °C, vorzugsweise bei Temperaturen zwischen 0 °C und 100 °C.

Zur Durchführung des erfindungsgemäßen Verfahrens (c) setzt man pro Mol an Pyridin-4-carbonsäureanilid der Formel (Ib) im allgemeinen 1.0 bis 5.0 Mol, vorzugsweise 1.0 bis 1.5 Mol an Alkylierungsmittel der Formel (IV) und gegebenenfalls 1.0 bis 3.0 Mol, vorzugsweise 1.0 bis 1.2 Mol an Reaktionshilfsmittel ein.
Die Reaktionsdurchführung, Aufarbeitung und Isolierung der Reaktionsprodukte erfolgt nach allgemein üblichen Methoden (vgl. z.B. JP 60/204 764).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff oder Ether, wie Diethylether, Dioxan, Tetrahydrofuran oder Ethylenglykoldimethyl- oder -diethylether.

Das erfindungsgmäße Verfahren (d) wird vorzugsweise in Gegenwart eines geeigneten Reaktionshilfsmittels durchgeführt. Als solche kommen vorzugsweise alle üblicher weise verwendbaren anorganischen oder organischen Säuren oder andere übliche Katalysatoren infrage.
Mit besonderem Vorzug verwendet man verdünnte wässrige oder konzentrierte Mineralsäuren, wie Salzsäure, Schwefelsäure oder Phosphorsäure, organische Sulfonsäuren, wie Methansulfonsäure oder p-Toluolsulfonsäure oder Säurechloride, wie z.B. Thionylchlorid, gegebenenfalls in Gegenwart eines Säurebindemittels, wie Pyridin oder Triethylamin.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 200 °C, vorzugsweise bei Temperaturen zwischen 20 °C und 150 °C.

Zur Durchführung des erfindungsgemäßen Verfahrens (d) setzt man pro Mol an Pyridin-4-carbonsäureanilid der Formel (Ia) im allgemeinen 1.0 bis 30.0 Mol, vorzugsweise 2.0 bis 5.0 Mol an Alkohol oder 1,0 bis 5,0 Mol an Diol der Formel (V) und gegebenenfalls 0.01 bis 2.0 Mol, vorzugsweise 0.1 bis 1.0 Mol an Reaktionshilfsmittel ein.
Die Reaktionsdurchführung, Aufarbeitung und Isolierung der Reaktionsprodukte erfolgt nach allgemein üblichen Methoden.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. Graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;

Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger der Braunfleckigkeit des Weizens (Leptosphaeria nodorum) oder gegen den Erreger der Braunfleckigkeit der Gerste (Pyrenophora teres) oder zur Bekämpfung von Krankheiten im Obst- und Gemüseanbau, wie beispielsweise gegen den Erreger des Apfelschorfes (Venturia (inaequalis) oder gegen den Erreger des Apfelmehltaus (Podosphaera leucotricha) oder gegen den Erreger des Gurkenmehltaus (Sphaerotheca fuliginea) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark po lare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low- Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

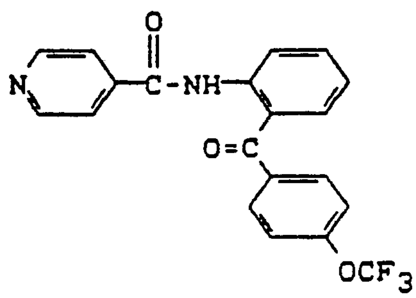

(Verfahren a)

Zu 14 g (0.05 Mol) 2-(4-Trifluormethoxybenzoyl)-anilin in 200 ml Pyridin gibt man portionsweise unter Rühren 9.8 g (0.055 Mol) Pyridin-4-carbonsäurechlorid Hydrochlorid, erhitzt anschließend 4 Stunden auf 50 °C, engt im Vakuum ein, nimmt den Rückstand in Wasser auf, extrahiert mehrfach mit Essigester, wäscht die vereinigten organischen Phasen mit Wasser, trocknet über Natriumsulfat und entfernt das Lösungsmittel im Vakuum.

Man erhält 13,6 g (71% der Theorie) an N-[2-(4-Trifluormethoxybenzoyl)-phenyl]-pyridin-4-carbonsäureamid vom Schmelzpunkt 112 - 114 °C.

## Herstellung der Ausgangsverbindung

### Beispiel III-1

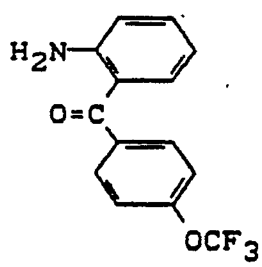

Zu 500 ml (0.5 Mol) einer 1normalen Bortrichloridlösung in Dichlormethan gibt man bei 0 °C bis 10 °C unter Rühren und Eiskühlung zunächst 45,4 ml (0.5 Mol) Anilin und 500 ml 1,2-Dichlorethan und anschließend abwechselnd portionsweise 102,8 g (0.55 Mol) 4-Trifluormethoxybenzonitril (vgl. z.B. J. org.

Chem. 44, 2907-2910 [1979]) und 66,6 g (0.5 Mol) Aluminiumtrichlorid. Nach beendeter Zugabe wird mit weiteren 500 ml 1,2-Dichlorethan verdünnt, Dichlormethan abdestilliert und 6 Stunden bei Rückfluß des 1,2-Dichlorethans (83 °C) gekocht. Anschließend wird auf Raumtemperatur abgekühlt, mit 400 ml 2normaler Salzsäure und weiteren 500 ml 1,2-Dichlorethan versetzt und weitere 3 Stunden bei Rückflußtemperatur gekocht. Nach dem Abkühlen erhält man durch Abfiltrieren und Trocknen 98 g (70 % der Theorie) an 2-(4-Trifluormethoxybenzoyl)-anilin als Feststoff. $^1$H-NMR ($CDCl_3$/Tetramethylsilan): $\delta$ = 7,2-7,4;
7,5-7,6;
7,7-7,8 ppm.

Beispiel 2

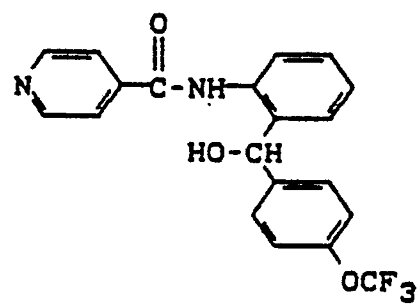

(Verfahren b)

Zu 10 g (0.026 Mol) N-[2-(4-Trifluormethoxybenzoyl)-phenyl]-pyridin-4-carbonsäureamid in 200 ml Methanol gibt man 1 g (0.026 Mol) Natriumborhydrid und rührt 15 Stunden bei Raumtemperatur. Zur Aufarbeitung wird im Vakuum eingeengt, der Rückstand mit Wasser versetzt, mehrfach mit Essigester extrahiert, die vereinigten Essigesterphasen über Natriumsulfat getrocknet und im Vakuum vom Lösungsmittel befreit.

Man erhält 9,4 g (93 % der Theorie) an N-[2-(4-Trifluormethoxy-α-hydroxybenzyl)-phenyl]-pyridin-4-carbonsäureamid vom Schmelzpunkt 65 °C.

Anwendungsbeispiele

In den folgenden Anwendungsbeispielen wurden die nachstehend aufgeführten Verbindungen als Vergleichsubstanzen eingesetzt:

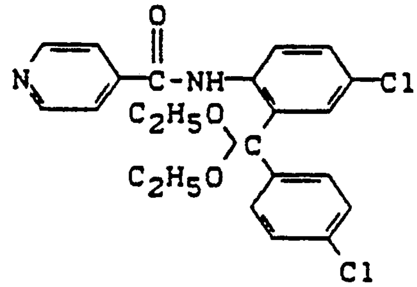

(A)

N-[4-Chlor-2-(4-chlor-α,α-diethoxybenzyl)-phenyl]-pyridin-4-carbonsäureamid

(bekannt aus EP 122 410)

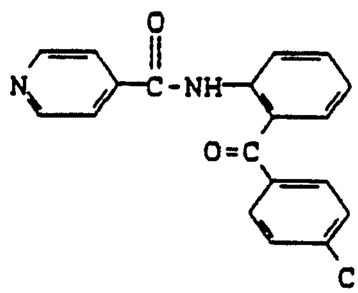

(B)

N-[2-(4-Chlorbenzoyl)-phenyl]-pyridin-4-carbonsäureamid

(bekannt aus Jp 60/23 364)

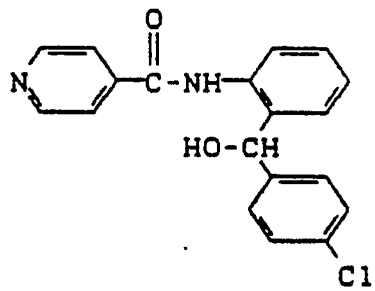

(C)

**N-[2-(4-Chlor-α-hydroxybenzyl)-phenyl]-pyridin-4-carbonsäureamid**

**(bekannt aus Jp 60/32 703)**

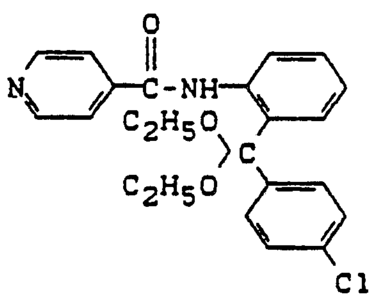

(D)

**N-[2-(4-Chlor-α,α-diethoxybenzyl)-phenyl]-pyridin-4-carbonsäureamid**

**(bekannt aus EP 122 410).**

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem

Test z.B. die Verbindungen gemäß den Herstellungsbeispielen 1 und 2.

Beispiel B

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1.

Beispiel C

Pyrenophora teres-Test (Gerste) / protektiv

Lösungsmittel: 100 Gewichtsteile Dimethylformamid
Emulgator: 0,025 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigt bei diesem Test z.B. die Verbindung gemäß dem Herstellungsbeispiel 1.

Beispiel D

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und

100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 1.

Beispiel E

Podosphaera-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23 °C und einer relativen Luftfeuchtigkeit von ca. 70 % aufgestellt.

9 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel 1.

## Ansprüche

1. Pyridin-4-carbonsäureanilide der allgemeinen Formel (I),

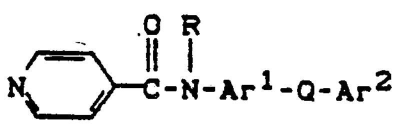

(I)

in welcher
R für Wasserstoff oder Alkyl steht,
Q für einen Rest der Formel

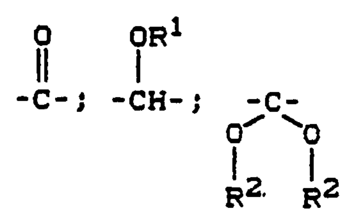

oder

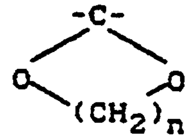

steht,
wobei
$R^1$ für Wasserstoff oder Alkyl steht,
$R^2$ für Alkyl steht und
n für eine Zahl 2, 3 oder 4 steht,
$Ar^1$ für einen gegebenenfalls substituierten o-Phenylenrest steht und
$Ar^2$ für gegebenenfalls substituiertes Phenyl steht,
mit der Maßgabe, daß mindestens einer der Reste $Ar^1$ oder $Ar^2$ substituiert ist durch Halogenalkoxy oder Halogenalkylthio, sowie deren Säureadditionssalze und Metallsalzkomplexe.

2. Pyridin-4-carbonsäureanilide gemäß Anspruch 1, wobei in der Formel (I)
R für Wasserstoff oder für geradkettiges oder verzweigtes Alkyl mit 1 bis 4 Kohlenstoffatomen steht,
Q für einen Rest der Formel

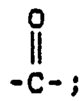 ; 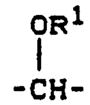 ; 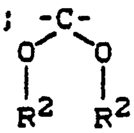

oder

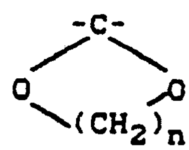

steht,
wobei
$R^1$ für Wasserstoff oder für geradkettiges oder verzweigtes Alkyl mit 1 bis 4 Kohlenstoffatomen steht,
$R^2$ für geradkettiges oder verzweigtes Alkyl mit 1 bis 4 Kohlenstoffatomen steht und
n für eine Zahl 2, 3 oder 4 steht,
$Ar^1$ für einen gegebenenfalls einfach bis vierfach, gleich oder verschieden substituierten o-Phenylenrest steht und
$Ar^2$ für gegebenenfalls einfach bis fünffach, gleich oder verschieden substituiertes Phenyl steht,
wobei als Phenyl- bzw. Phenylensubstituenten für die Reste $Ar^1$ bzw. $Ar^2$ jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,
mit der Maßgabe, daß mindestens einer der Reste $Ar^1$ oder $Ar^2$ substituiert ist durch Halogenalkoxy oder Halogenalkylthio und deren Säureadditionssalze und Metallsalzkomplexe.

3. Pyridin-4-carbonsäureanilide gemäß Anspruch 1, wobei in der Formel (I),
R für Wasserstoff, Methyl oder Ethyl steht,
Q für einen Rest der Formel

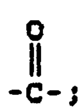 ; 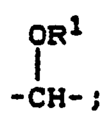 ; 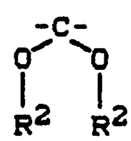

oder

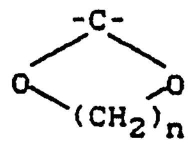

steht,
wobei
$R^1$ für Wasserstoff, Methyl oder Ethyl steht,
$R^2$ für Methyl oder Ethyl steht und
n für eine Zahl 2 oder 3 steht,
$Ar^1$ für einen gegebenenfalls ein- bis dreifach, gleich oder verschieden substituierten o-Phenylenrest steht und
$Ar^2$ für gegebenenfalls ein- bis dreifach, gleich oder verschieden substituiertes Phenyl steht, wobei als Phenyl- bzw. Phenylensubstituenten für die Reste $Ar^1$ bzw. $Ar^2$ jeweils infrage kommen:
Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Trifluormethyl, Trifluormethoxy, Trifluormethylthio, Difluormethyl, Difluormethoxy, Difluormethylthio, Difluorchlormethyl, Difluorchlormethoxy, Difluorchlormethylthio, Dichlorfluormethyl, Dichlorfluormethoxy oder Dichlorfluormethylthio,
mit der Maßgabe, daß mindestens einer der Reste $Ar^1$ oder $Ar^2$ substituiert ist durch Trifluormethoxy, Trifluormethylthio, Difluormethoxy, Difluormethylthio, Difluorchlormethoxy, Difluorchlormethylthio, Dichlorfluormethoxy oder Dichlorfluormethylthio, und deren Säureadditionssalze und Metallsalzkomplexe.

4. Pyridin-4-carbonsäureanilide gemäß Anspruch 1, wobei in der Formel (I)
R für Wasserstoff oder Methyl steht,
Q für einen Rest der Formel

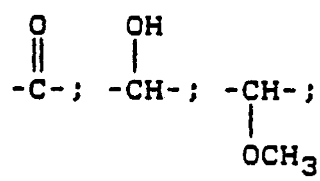

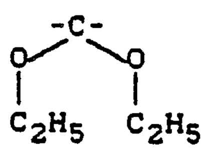

oder

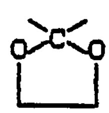

steht,
$Ar^1$ für einen gegebenenfalls ein- bis dreifach, gleich oder verschieden substituierten o-Phenylenrest steht und
$Ar^2$ für gegebenenfalls ein- bis dreifach, gleich oder verschieden substituiertes Phenyl steht, wobei als Phenyl- bzw. Phenylensubstituenten für die Reste $Ar^1$ bzw. $Ar^2$ jeweils infrage kommen: Fluor, Chlor, Methyl, Trifluormethyl, Trifluormethoxy, Difluormethoxy oder Difluorchlormethoxy
mit der Maßgabe, daß mindestens einer der Reste $Ar^1$ oder $Ar^2$ substituiert ist durch Trifluormethoxy, Difluormethoxy oder Difluorchlormethoxy und deren Säureadditionssalze und Metallsalzkomplexe.

5. Verfahren zur Herstellung von Pyridin-4-carbonsäureaniliden der Formel (I),

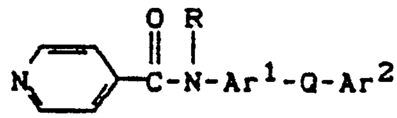 (I)

in welcher
R für Wasserstoff oder Alkyl steht,
Q für einen Rest der Formel

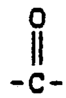 ; 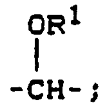 ; 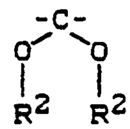

oder

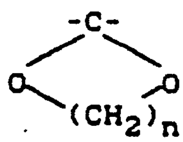

steht,
wobei
$R^1$ für Wasserstoff oder Alkyl steht,
$R^2$ für Alkyl steht und
n für eine Zahl 2, 3 oder 4 steht,
$Ar^1$ für einen gegebenenfalls substituierten o-Phenylenrest steht und
$Ar^2$ für gegebenenfalls substituiertes Phenyl steht,
mit der Maßgabe, daß mindestens einer der Reste $Ar^1$ oder $Ar^2$ substituiert ist durch Halogenalkoxy oder Halogenalkylthio, sowie deren Säureadditionssalze und Metallsalzkomplexe, dadurch gekennzeichnet, daß man, um

(a) Pyridin-4-carbonsäureanilide der Formel (Ia),

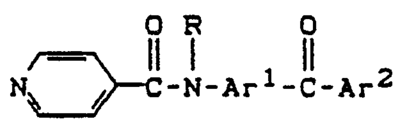 (Ia)

in welcher
R, $Ar^1$ und $Ar^2$ die oben angegebene Bedeutung haben, zu erhalten,
ein Pyridin-4-carbonsäurehalogenid Hydrochlorid der Formel (II),

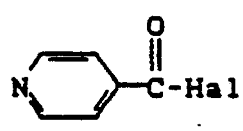 (II)

in welcher
Hal für Halogen steht,

mit Aminobenzophenonen der Formel (III),

R-NH-$Ar^1$-$\overset{O}{\overset{\|}{C}}$-$Ar^2$ (III)

in welcher

R, $Ar^1$ und $Ar^2$ die oben angegebene Bedeutung haben,

gegebenenfalls in Gegenwart eines Verdünnungsmittels und gegebenenfalls in Gegenwart eines Säurebindemittels umsetzt; oder um

(b) Pyridin-4-carbonsäureanilide der Formel (Ib),

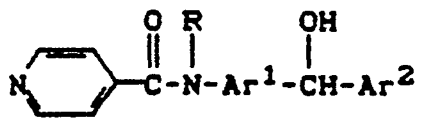 (Ib)

in welcher

R, $Ar^1$ und $Ar^2$ die oben angegebene Bedeutung haben, zu erhalten,

die mit Hilfe des Verfahrens (a) erhältlichen Pyridin-4-carbonsäureanilide der Formel (Ia),

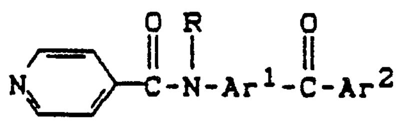 (Ia)

in welcher

R, $Ar^1$ und $Ar^2$ die oben angegebene Bedeutung haben,

mit Reduktionsmitteln gegebenenfalls in Gegenwart eines Verdünnungsmittels umsetzt; oder um

(c) Pyridin-4-carbonsäureanilide der Formel (Ic),

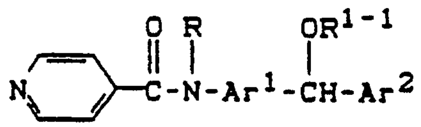 (Ic)

in welcher

$R^{1-1}$ für Alkyl steht und

R, $Ar^1$ und $Ar^2$ die oben angegebene Bedeutung haben, zu erhalten,

die mit Hilfe des Verfahrens (b) erhältlichen Pyridin-4-carbonsäureanilide der Formel (Ib),

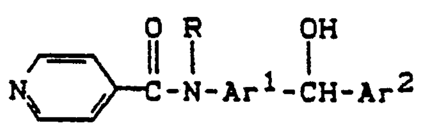 (Ib)

in welcher

R, $Ar^1$ und $Ar^2$ die oben angegebene Bedeutung haben,

mit Alkylierungsmitteln der Formel (IV),

$R^{1-1}$-E (IV)

in welcher

$R^{1-1}$ die oben angegebene Bedeutung hat und

E für eine elektronenanziehende Abgangsgruppe steht,

gegebenenfalls in Gegenwart eines Verdünnungsmittels und gegebenenfalls in Gegenwart eines Reaktionshilfsmittels umsetzt;

(d) oder um Pyridin-4-carbonsäureanilide der Formel (Id),

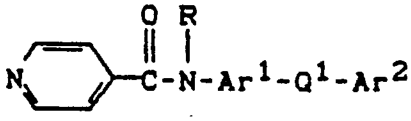

(Id)

in welcher
$Q^1$ für einen Rest der Formel

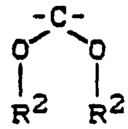

oder

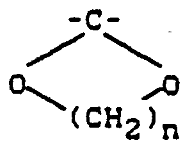

steht, wobei
$R^2$ und n die oben angegebene Bedeutung haben, zu erhalten,
die mit Hilfe des Verfahrens (a) erhältlichen Pyridin-4-carbonsäureanilide der Formel (Ia),

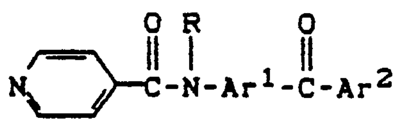

(Ia)

in welcher
R, $Ar^1$ und $Ar^2$ die oben angegebene Bedeutung haben,
mit mindestens 2 Mol Alkohol oder mindestens 1 Mol Diol der Formel (V),
$HO-R^3$ (V)
in welcher
$R^3$ für Alkyl oder für einen Rest $-(CH_2)_n-OH$ steht,
wobei
n die oben angegebene Bedeutung hat,
gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

6. Schädlingsbekämpfungsmittel, gekennzeichnet durch einen Gehalt an mindestens einem Pyridin-4-carbonsäureanilid der Formel (I) nach den Ansprüchen 1 oder 5.

7. Verwendung von Pyridin-4-carbonsäureanilide der Formel (I) nach den Ansprüchen 1 oder 5 zur Bekämpfung von Schädlingen.

8. Verfahren zur Bekämpfung von Schädlingen, dadurch gekennzeichnet, daß man Pyridin-4-carbonsäureanilide der Formel (I) nach den Ansprüchen 1 oder 5 auf Schädlinge und/oder ihren Lebensraum einwirken läßt.

9. Verfahren zur Herstellung von Schädlingsbekämpfungsmitteln, dadurch gekennzeichnet, daß man Pyridin-4-carbonsäureanilide der Formel (I) nach den Ansprüchen 1 oder 5 mit Streckmitteln und/oder oberflächenaktiven Mitteln vermischt.